# EUROPEAN PATENT SPECIFICATION

(11) **EP 1 611 247 B1**
(45) Date of publication and mention of the grant of the patent: **08.10.2008**
(21) Application number: 04725734.0
(22) Date of filing: 05.04.2004
(51) Int. Cl.: C12Q 1/26, C12N 9/02, C12N 15/00, C12Q 1/68, C07K 16/00, A61K 48/00, A61K 38/00, C12N 5/10, G01N 33/50

(54) **CYP2S1 AS TARGET FOR DIAGNOSIS AND THERAPY OF SKIN DISEASES**
CYP2S1 ALS ZIEL FÜR DIE DIAGNOSE UND THERAPIE VON HAUT KRANKHEITEN
CYP2S1 COMME CIBLE POUR LE DIAGNOSTIC ET LA THERAPIE DE MALADIES DE LA PEAU

(30) Priority: 05.04.2003 GB 0307914
(43) Date of publication of application: 04.01.2006
(73) Proprietor: The University Court of the University of Dundee, Dundee DD1 4HN (GB)
(72) Inventor: SMITH, Gillian, Dundee DD2 3FF (GB); IBBOTSON, Sally, Helen, Dundee DD1 9SY (GB); WOLF, Charles Roland, Inchture PH14 9QS (GB)
(74) Representative: Chapman, Paul Gilmour
(86) International application number: PCT/GB2004/001453
(87) International publication number: WO 2004/091150

(56) References cited:
- WO-A-01/79468
- WO-A-03/101376
- RIVERA STEVEN P ET AL: "Identification of a novel dioxin-inducible cytochrome P450" MOLECULAR PHARMACOLOGY, vol. 61, no. 2, February 2002 (2002-02), pages 255-259, XP002295652 ISSN: 0026-895X cited in the application
- FURUKAWA M ET AL: "Adenovirus vector-mediated reporter system for in vivo analyses of human CYP3A4 gene activation" JOURNAL OF BIOCHEMISTRY, JAPANESE BIOCHEMICAL SOCIETY, TOKYO, JP, vol. 131, no. 1, January 2002 (2002-01), pages 71-78, XP002969418 ISSN: 0021-924X
- RYLANDER TOVE ET AL: "Identification and tissue distribution of the novel human cytochrome P450 2S1 (CYP2S1)" BIOCHEMICAL AND BIOPHYSICAL RESEARCH COMMUNICATIONS, vol. 281, no. 2, 23 February 2001 (2001-02-23), pages 529-535, XP002295653 ISSN: 0006-291X cited in the application
- DATABASE EMBL [Online] EBI; 23 October 2000 (2000-10-23), DOE JOINT GENOME INSTITUTE: XP002295655 retrieved from EBI Database accession no. AC011510
- SMITH G ET AL: "Cutaneous expression of cytochrome P450 CYP2S1: individuality in regulation by therapeutic agents for psoriasis and other skin diseases" LANCET THE, LANCET LIMITED. LONDON, GB, vol. 361, no. 9366, 19 April 2003 (2003-04-19), pages 1336-1343, XP004421152 ISSN: 0140-6736
- SMITH G ET AL: "Cytochrome P450 CYP2S1 expression in human skin: Individuality in regulation by therapeutic agents for psoriasis and other skin diseases." BRITISH JOURNAL OF DERMATOLOGY, vol. 148, no. 4, April 2003 (2003-04), page 853, XP002295654 & ANNUAL MEETING OF THE BRITISH SOCIETY FOR INVESTIGATIVE DERMATOLOGY; SOUTHAMPTON, UK; APRIL 07-09, 2003 ISSN: 0007-0963

## Description

### Field of the Invention

This invention relates to a retinoic acid metabolizing cytochrome P450 2S1 (CYP2S1) and to uses of sequence specific nucleic acid, primers and antibodies, in, for example, assays for the analysis of CYP2S1 messenger RNA and/or protein expression in skin.

### Background of the Invention

Inter-individual differences in response to topical drug treatment and photo(chemo)therapy are a significant clinical problem in the treatment of common skin diseases such as psoriasis. Individuality in hepatic drug metabolising enzyme expression is an important determinant of systemic drug handling; similar variation in cutaneous gene expression may therefore contribute to individuality in response to topical therapies.

Human skin is the largest organ of the body. In addition to its role as a physical barrier, there is increasing evidence that skin is metabolically active. For example, it has recently been reported that human skin expresses a diverse and functional cytochrome P450 (CYP450) monooxygenase system¹⁻⁴. CYP450s have been extensively studied in other organs, particularly liver, where they are essential catalysts of drug and xenobiotic metabolism. There is marked inter-individual variation in the expression and activity of many hepatic CYP450s, resulting in inter-individual differences in response to drug therapies and in the incidence of adverse drug reactions⁵.

Skin disease is common, with one third of the population affected at any one time⁶. Psoriasis affects approximately 2% of the population and is a major cause of morbidity and a significant therapeutic challenge. Inter-individual differences in response to topical drug treatment and ultraviolet radiation (UVR) in the treatment of common skin disorders, such as psoriasis, are unpredictable. While the majority of patients tolerate drug therapy, a proportion develop adverse effects or are "treatment-resistant". Similarly, it is not possible to predict which patients will respond rapidly and well to ultraviolet B (UVB) phototherapy or psoralen-ultraviolet A (PUVA) photo(chemo)therapy or those who will be less responsive. An understanding of the factors which determine how individual patients respond to drug or UVR therapies may enable the outcome of treatment to be more predictable, thus reducing side effects and facilitating the selection of optimal treatment regimens on an individual basis.

One factor which may contribute to variation in response to treatment is inter-individual differences in the cutaneous expression of drug metabolising enzymes and cytoprotective genes. Human skin is the first line of defence against environmental insult and it seems logical that it would be a rich source of cytoprotective genes, thought to have evolved as an adaptive response to environmental challenge⁷. Relatively little is known about the expression and regulation of these genes in human skin although they have been extensively studied in liver and in rodent and cell culture models.

CYP450s are a multigene family of Phase I monooxygenases which, together with their redox partner NADPH cytochrome P450 reductase (CPR), catalyse the oxidative metabolism of the vast majority of drugs and xenobiotics to which we are exposed⁸. CYP450s in subfamilies CYP1 to CYP4 are responsible for the majority of foreign compound metabolism, have unique but overlapping substrate specificities and, in general, are regulated by substrate-induced activation of gene transcription⁹. CYP450 expression is highest in the liver, but many CYP450s are also expressed at significant levels in extra-hepatic tissues¹⁰. CYP450 expression was first described in human skin more than 20 years ago¹¹ and was shown to be inducible following the topical application of crude coal tar, a source of polycyclic aromatic hydrocarbons (PAHs), known to be potent inducers of CYP450 gene transcription¹². Constitutive expression of CYP1A1 and the closely related isozyme CYP1B1 in human keratinocytes was recently confirmed by RT-PCR in a series of experiments which also identified mRNAs encoding CYP2B6, CYP2E1 and CYP3A5¹. In a complementary study, mRNAs encoding CYP450s CYP2A6, CYP2B6 and CYP3A4 were identified in primary human keratinocytes using RNase protection analysis². Interestingly, recent data suggests that Ultaviolet B (UVB) irradiation can induce the cutaneous expression of CYP1A1, CYP1B1³ and CYP4A11⁴. An increased understanding of cutaneous gene expression and regulation by topical chemicals and photo(chemo)therapy and the ability to determine individual patient phenotypes may lead to a more rational approach to the treatment of common skin diseases.

It is an object of the present invention to obviate and/or mitigate at least one of the aforementioned disadvantages.

It is a further object of the present invention to provide means to detect aberrant CYP450 expression in diseased skin and to use this information to design the most appropriate therapy for a patient.

The present invention is based in part on the observations by the present inventors that the cytochrome P450-GXP2S1 is expressed in human skin to different degrees between healthy and diseased individuals.

CYP2S 1, has been identified from human genome databases. This enzyme is primarily expressed in extra-hepatic tissues including the trachea, lung and small intestine¹³. CYP2S1 has been localised to chromosome 19q13·2, in a cluster with other GYP2 family CYP450s which are known to be involved in drug and xenobiotic metabolism¹⁴. Using quantitative real-time RT-PCR, the present inventors have investigated CYP2S1 expression in human skin from healthy volunteers and psoriasis patients and demonstrated its regulation by UVR and PUVA, and by specific topical drugs used in the treatment of psoriasis. The inventors have additionally investigated whether there are inter-individual differences in constitutive CYP2S1 expression and inducibility by UVR and drug treatment.

### Summary of the invention

According to a first aspect of the present invention there is provided a method for identifying an agent capable of modulating expression of CYP2S1 by a cell, in accordance with claim 1. Other aspects of the present invention are recited in independent claims 2, 12, 15-19. Preferred features of certain embodiments of the present invention are defined in the claims which are dependent on the aforementioned independent claims.

The present invention relates to CYP2S1 expression in skin tissue and applications resulting there from. In general, the present invention relates to the identification of novel treatments for skin diseases, as well as the identification of patients displaying aberrant CYP2S 1 expression and/or determining the effectiveness or otherwise of any skin treatment regime. Any skin condition which is associated with an increase or decrease of CYP2S 1 expression may be the subject of the present invention. Thus, based on the information provided herein, a skilled man may identify other skin conditions which may result in an increase and/or disease in CYP2S1 expression in skin tissue. However, of particular note is the treatment of psoriasis.

It is to be understood that the term "modulates" refers to both increased and decreased expression and/or activity of CYP2S1. Moreover, the term "expression" refers to mRNA expression and/or protein expression. The term "activity" as used herein, may be related to an increase or decrease in expression of CYP2S1, but can conceivably be an increase or decrease in activity due to stabilisation or destabilisation of CYP2S1, or other increased activity not associated with merely an increase in the amount of enzyme.

### Detailed Description of the Intention

Polypeptide component CYP2S1 refers to the CYP2 protein, or a homologue thereof or a derivative thereof, wherein said homologues or derivatives are capable of metabolising the same substrates as the native CYP2S 1 protein. The CYP2S1 protein may be expressed from the cDNA sequence identified under the NCBI accession number NN_030622. Alternatively, this sequence may be used to identify homologues which are capable of hybridising to this sequence under high stringency conditions. The skilled man is directed to the teaching of Sambrook et al - Molecular Cloning: A Laboratory Manual (3 - Volume Set), 2001, Cold Spring Harbour Laboratory Press, for details of suitable hybridisation conditions as well as other molecular biology techniques referred to throughout this specification.

The present invention is particularly directed to the intended treatment of human subjects and therefore human skin, but this should not be construed as limiting, as it may also be appropriate to treat skin from other animals, in which case it may appropriate to use a CYP2S1 homologue obtained from the animal species being studied.

It will be understood that for particular polypeptides embraced herein, natural variations such as may occur due to polymorphism, can exist between individuals or between members of the family. These variations may be demonstrated by (an) amino acid difference(s) in the overall sequence or by deletions, substitutions, insertions, inversions or additions of (an) amino acid(s) in said sequence. All such derivatives showing the recognised CYP2S 1 activity are included within the scope of the invention.

Derivatives may be in the form of a fusion protein wherein CYP2S1, a homologue, or a derivative thereof is fused using standard cloning techniques to another polypeptide which may, for example, comprise a DNA binding domain, a transcriptional activation domain or a ligand suitable for affinity purification (for example glutathione-S-transferase or six consecutive histidene residues).

CYP2S 1 as used in the methods of the present invention may be obtained from skin tissue, or alternatively produced recombinantly from, for example, bacteria, yeast or higher eukaroytic cells including mammalian cell lines and insect cell lines, or synthesised de novo using commercially available synthesisers.

In certain embodiments it may be desirable to express CPR also.

### Test Agents

A test agent which modulates the expression and/or activity of CYP2S1 may do so in several ways. For example, the test agent may modulate the binding of CYF2S1 to its substrate and test agents of the type may conventionally be screened by *in vitro* binding assays as, for example, described below. Examples of such test agents include non-functional homologues of CYP2S1, as well as antibodies which specifically bind to CYP2S1.

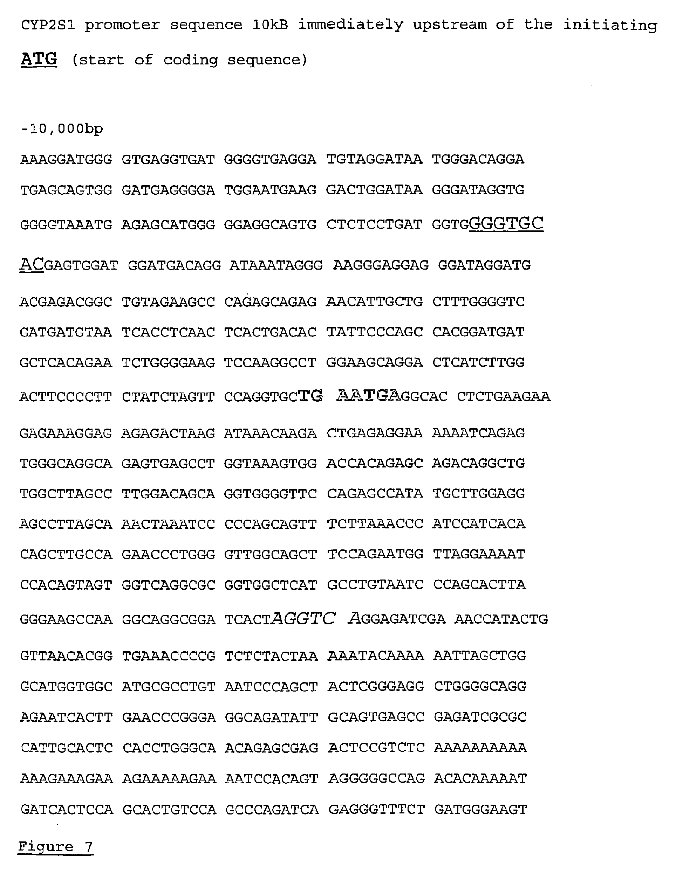

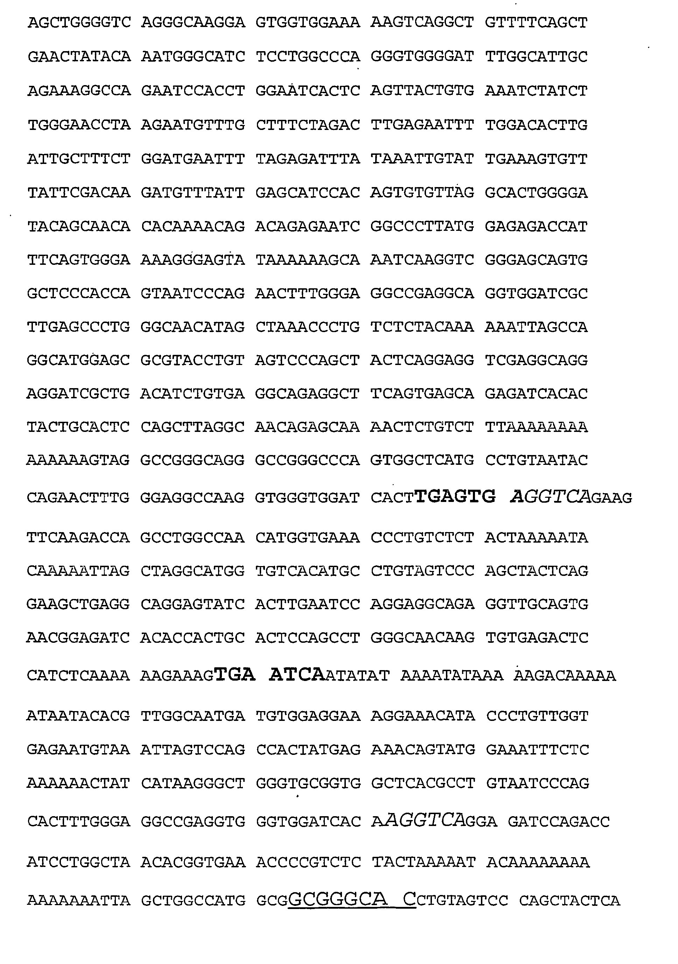

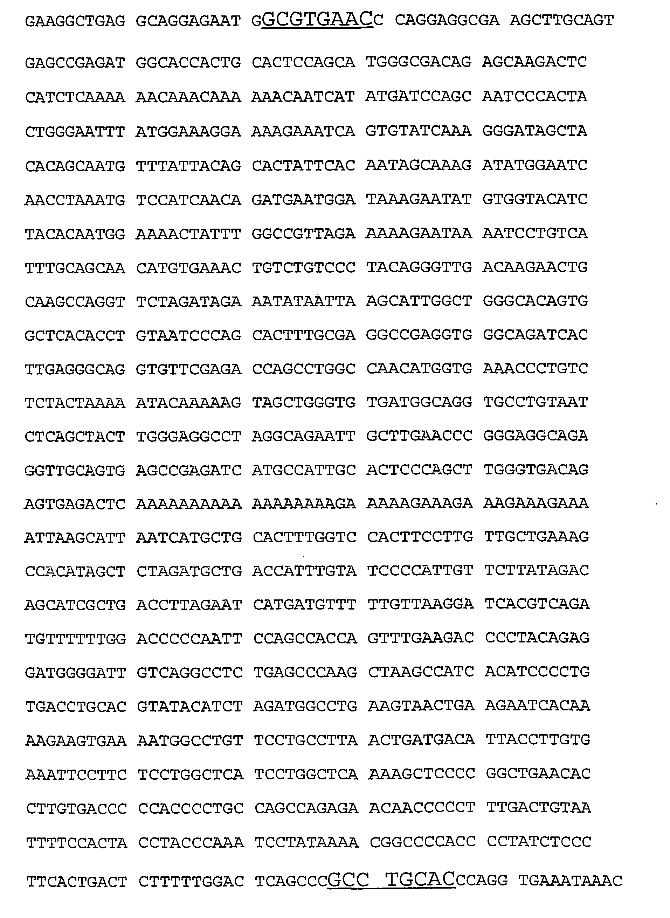

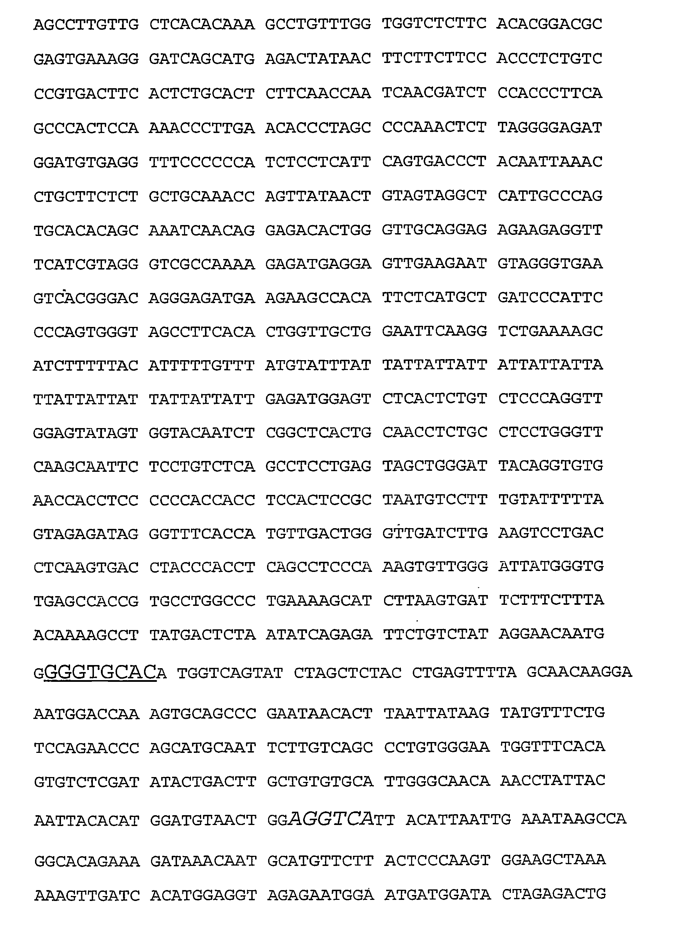

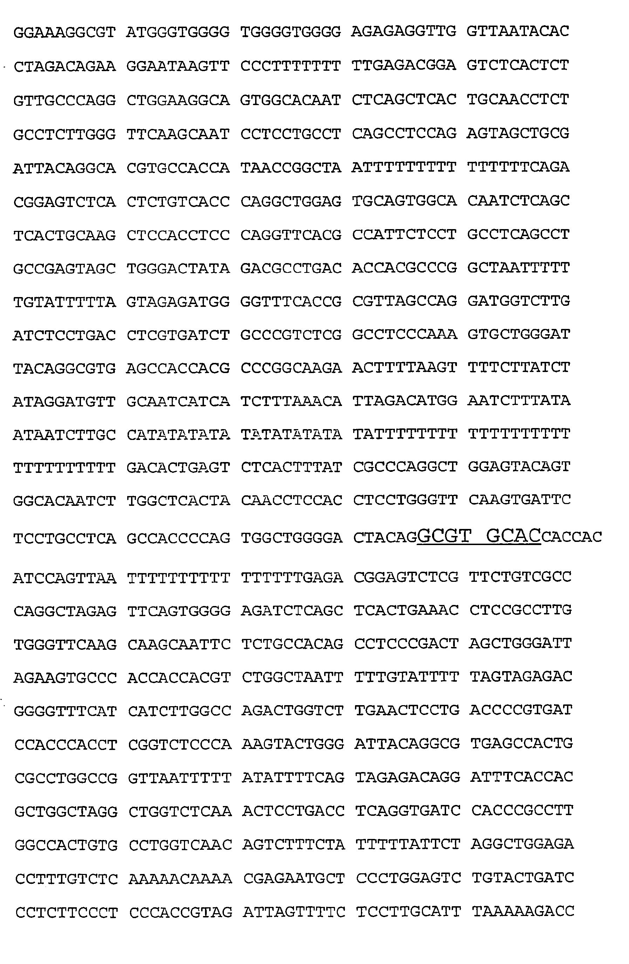

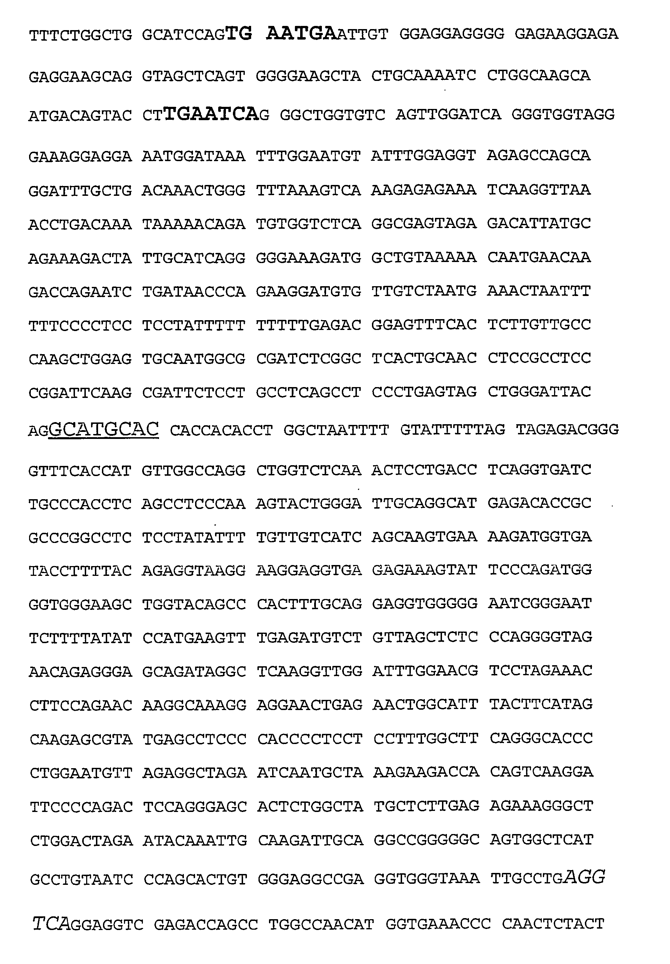

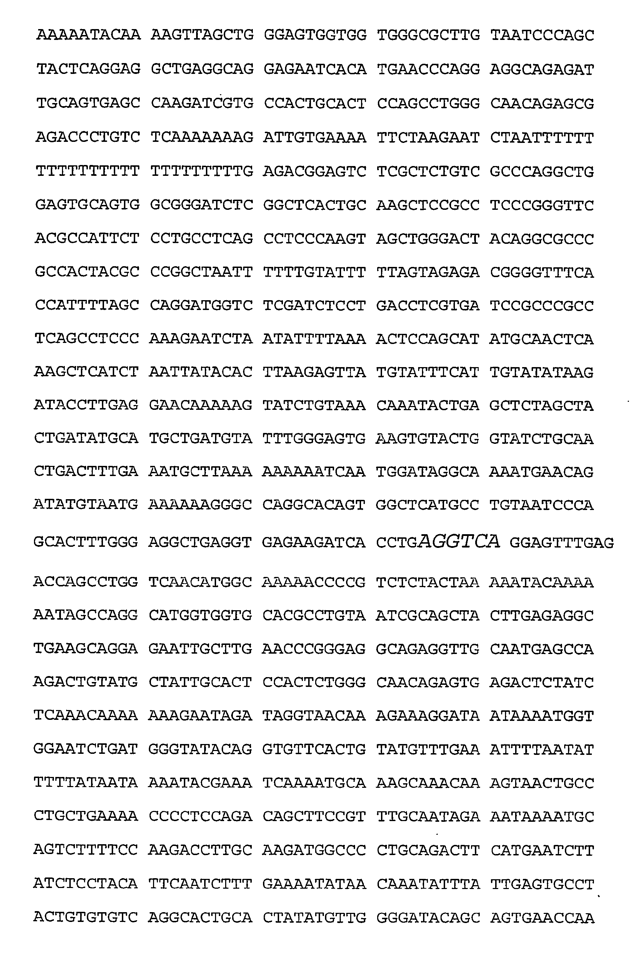

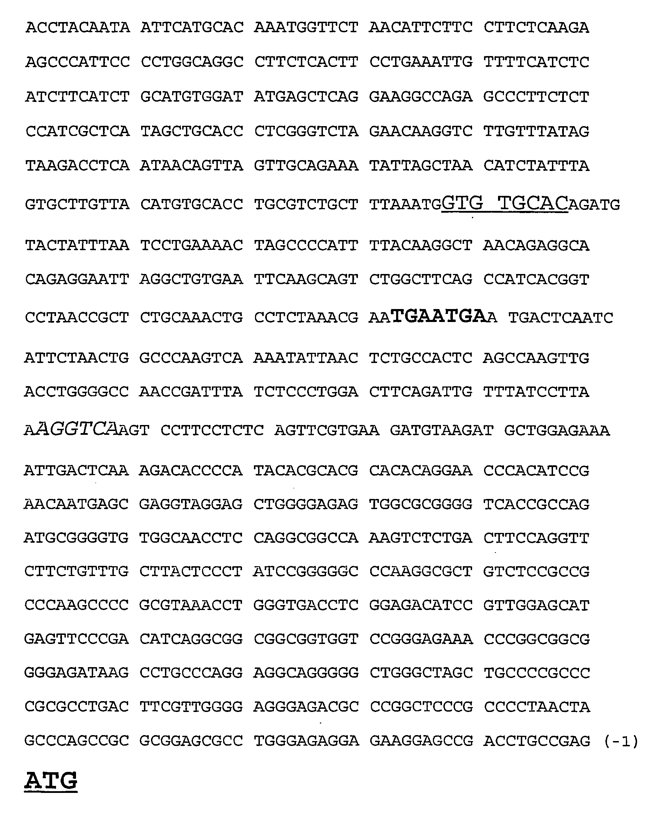

A test agent which has an effect on expression of CYP2S1, may do so by binding to the nucleic acid responsible for controlling CYP2S1 expression. Typically, such a test agent binds to regulatory sequences found upstream of the CYP2S1 gene sequence and the present invention provides a 10 kilo base region of such a sequence, in which a number of putative regulatory domains are identified. The 10 kilo base sequence shown in Figure 7 can be used to affect translation of CYP2S 1 or a suitable reporter protein known to those skilled in the art.. It is also relatively straightforward for the skilled man to produce deletion mutants of this sequence and test for their ability to still affect expression of CYP2S1 or a reporter protein.

Test agents, which are able to effect expression of CYP2S1 may include antisense oligonucleotides, RNAi molecules, chemical drug candidates, peptides or proteins, radiation, such as UV radiation, or radiation in combination with a chemical which is capable of being activated by said radiation. Suitable test agents may also include antibody products (for example, monoclonal and polyclonal antibodies, single chain antibodies, chimeric antibodies and CDR-grated antibodies), which are specific for CYP2S1.

Test agents may be used in an initial screen in batches of, for example 10 substances per reaction, and the substances of those batches which show modulation in CYP2S1 expression and/or activity, tested individually. Candidate substances which show activity in *in vitro* screens, such as those described below, can then be tested in *in vivo* systems, such as mammalian cells which are exposed to the modulatory test agent and tested for toxicity and/or usefulness in treating appropriate skin conditions.

Administration of candidate substances to cells, may be preformed by, for example, adding directly to the cell culture medium, or injection into the cell. It should be appreciated that references generally made to cell based assays, but it may be possible to carry out the methods using cell free based systems, known to the skilled addressee.

Where the test agent, is for example, radiation, such as UV radiation, this may be easily applied to the cell or cell culture medium. It should be appreciated that reference to cell and cell cultures also extends to the generation of so called "living-skin equivalents", which are known in the art and are architecturally similar to living-skin.

### Methods

The methods of the present invention may be *in vitro* assays or *in vivo* assays, for example using an appropriate animal model. For example, one type of *in vitro* binding assay may involve immobilising CYP2S1 on a solid support and thereafter contacting immobilised CYP2S1 with a test agent and determining if the test agent binds to CYP2S1.

Alternatively, the test agent may be immobilised and CYP2S1 non-immobilised. Binding of the test agent to CYP2S1 (and vice/versa) may be determined by a variety of methods well known in the art. For example, a detection agent may be labelled (with for example, a radio active label, an epidope tag or an enzyme-antibody conjugate). The effect of the test agent binding to CYP2S1 can be determined by comparing the amount of label bound in the presence of the test agent with the amount of label bound in the absence of the test agent. A lower amount of label bound in the presence of the test agent indicates that the test agent is able to bind to CYP2S1.

To determine whether or not a test agent is capable of modulating expression of CYP2S1, it is possible to detect for example the synthesis of CYP2S1 mRNA, or alternatively CYP2S1 protein. Detection of CYP2S1 mRNA can be carried out by a variety of techniques as disclosed in Sambrook et al, referenced above, and a particularly suitable method is the use quantitative real-time PCR analysis using CYP2S1 sequence specific primers and probes. Detection of CYP2S1 protein expression may be carried out for example, by western blotting techniques using an antibody specifically reactive to CYP2S1.

As an alternative to specifically detecting CYP2S1 expression, it is also possible to utilise the upstream transcription and/or translation regulatory sequences of CYP2S1, to control expression of a reporter protein. Again, this is well known to a skilled addressee and suitable techniques are disclosed in Sambrook et al and many constructs into which the upstream may be ligated, are known in the art.

### Testing Drug effectiveness and/or toxicity

The present invention also provides methods for testing the likely effectiveness of a particular skin treatment to a subject. For example, once a skin condition is identified as being regulated or associated with CYP2S1 expression, it may be appropriate to detected levels of CYP2S1 in the skin of the patient, prior, during and or after treatment. It may also be appropriate to carryout such tests in order to identify patients which display a polymorphism in the CYP2S1 gene and/or promoter sequences, as subjects which display such a polymorphism may display increased or reduced levels of CYP2S1, which may be taken into account when designing an appropriate therapeutic regime.

For example, patients which display reduced levels of CYP2S1 expression, when it is determined that such an expression would be beneficial in their treatment, can be given CYP2S1 in the form of a protein and/or a vector designed to express the protein in disease skin. Alternatively, where it is identified that a patient displays an increased level of CYP2S1 expression and such an increase is undesirable in treating a skin condition, it may be possible to add an agent which reduces activity of CYP2S1 and therefore improve subsequent treatment.

The present invention also allows the testing of current skin treatments, in order to ascertain if these are regulated and/or controlled by CYP2S1 expression and/or activity. For example, if it can be determined that a current treatment does act in such a manner, then it may be appropriate to screen individuals for their CYP2S1 expression and/or activity profile in order to ascertain whether or not a treatment is likely to be beneficial to them.

Moreover, when screening potential candidates, it may be appropriate to add such candidates to a system comprising CYP2S1, in order to determine any metabolites which may be produced through action of CYP2S1.

In summary, the present invention provides methods which may be used for the identification of modulators of CYP2S1 expression which are useful in the treatment of skin disorders such as psoriasis.

Another aspect of the invention is a method of modulating CYP2S1 expression where such modulation either increases or decreases metabolism of agents, such as *all trans* retinoic acids used in the treatment of skin disorders, in particular psoriasis.

Tests would also include transactivation assays using the CYP2S1 promoter. It may also be desirable to express CYP2S1 in skin for gene therapy.

The present invention will now be further described by way of non-limiting Example and with reference to the Figures which show:

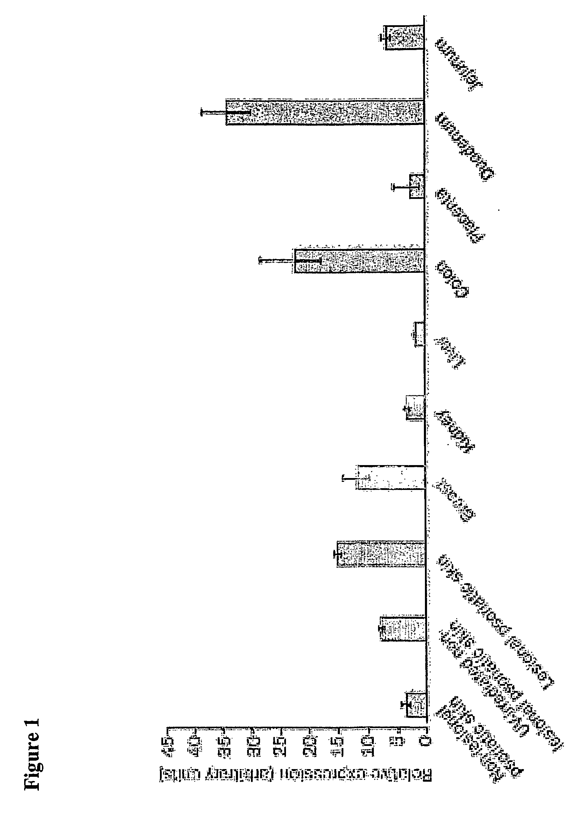

Figure 1 shows constitutive CYP2S1 mRNA expression in various human tissues. Quantitative real-time RT-PCR was used to compare CYP2S1 mRNA expression in a panel of human tissues including skin, breast, kidney, liver, placenta and regions of the gastrointestinal tract. CYP2S1 mRNA expression in UVR-treated non-lesional skin, in psoriatic plaque and in adjacent untreated control skin within the same subject is shown. Each of the other non-cutaneous tissue samples was obtained from a different individual and was analysed in triplicate. CYP2S1 expression was normalised to 18S ribosomal RNA to ensure equality of loading. Results are presented as the mean of triplicate determinations, ± standard error.

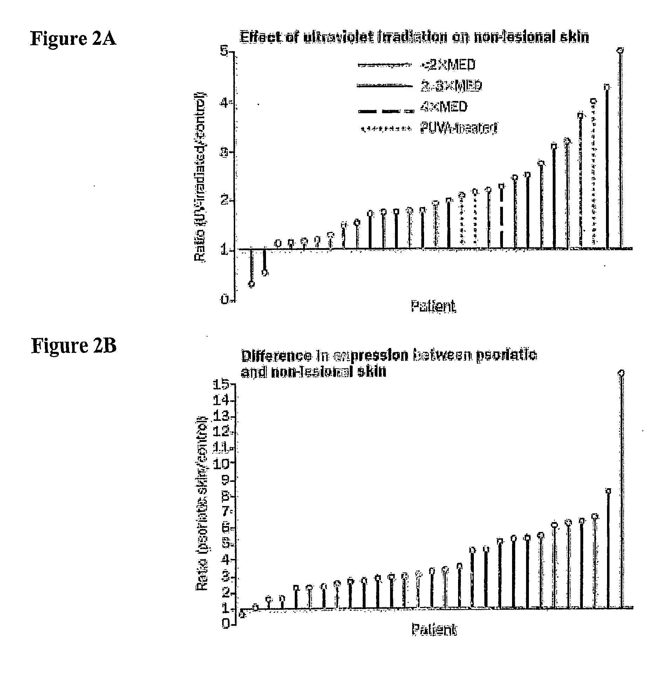

Figure 2 shows CYP2S1 mRNA expression in patients exposed to UVR and in psoriatic plaque versus non-lesional skin
   Each data point represents fold change in CYP2S1 expression in one patient. CYP2S1 mRNA expression in UVR irradiated non-lesional skin (MED = minimum erythema dose) compared with adjacent untreated non-lesional control skin in the same patients with psoriasis. Expression in PUVA-treated skin is also shown (Figure 2A). CYP2S1 mRNA expression in psoriatic plaque compared with adjacent untreated non-lesional control skin in the same patients (Figure 2B). Each data point represents fold change in CYP2S 1 expression in one patient.

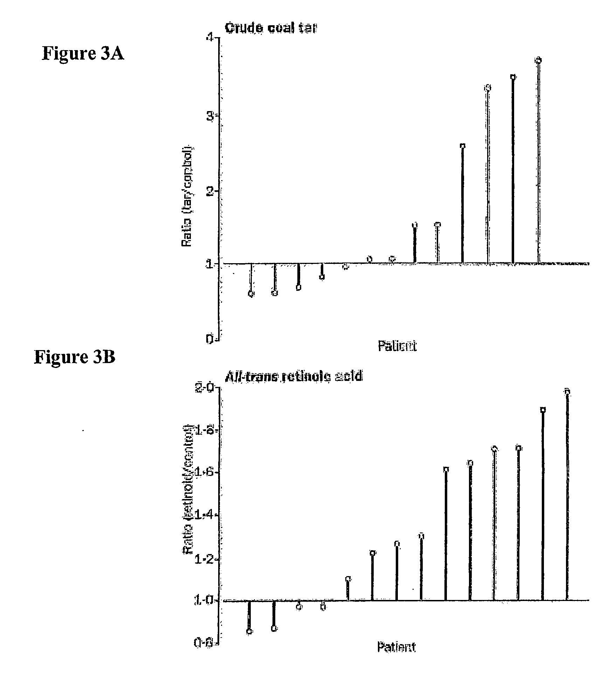

Figure 3 shows the change in CYP2S1 mRNA expression by topical chemicals CYP2S1 mRNA expression in healthy volunteers treated on photoprotected buttock sites with either crude coal tar (12% (w/w) in petrolatum) (n=13) (Figure 3A) or *all trans* retinoic acid (0-025% (w/w) RetinA^{®} gel) (n=14) (Figure 3B). Data represent gene expression in chemically-treated skin compared to adjacent untreated control skin expression in the same individuals. Each data point represents fold change in CYP2S1 expression in one subject.

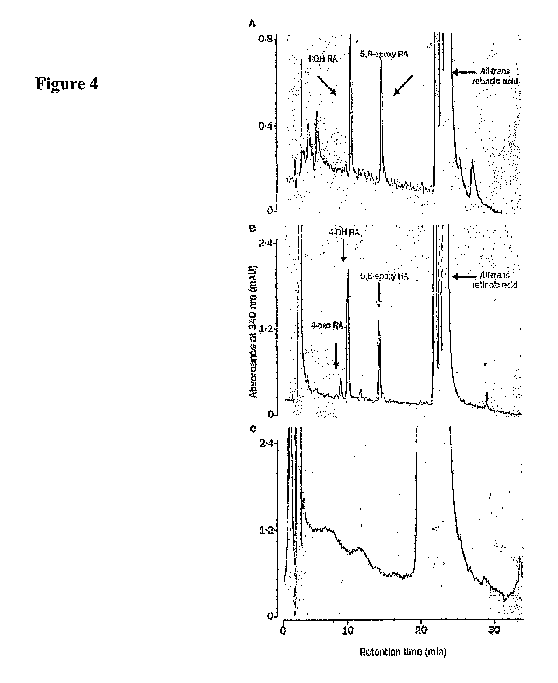

Figure 4 shows the metabolism of atRA by CYP2S 1 *E. Coli* membranes co-expressing CYP2S1 and CPR were incubated with atRA as previously described¹⁷. CYP2S1-derived atRA metabolites (Figure 4A) were separated by HPLC and identified by comparison with the elution profile of human liver microsomes (Figure 4B). Control incubations were performed in the absence of the NADPH regenerating system (Figure 4C).

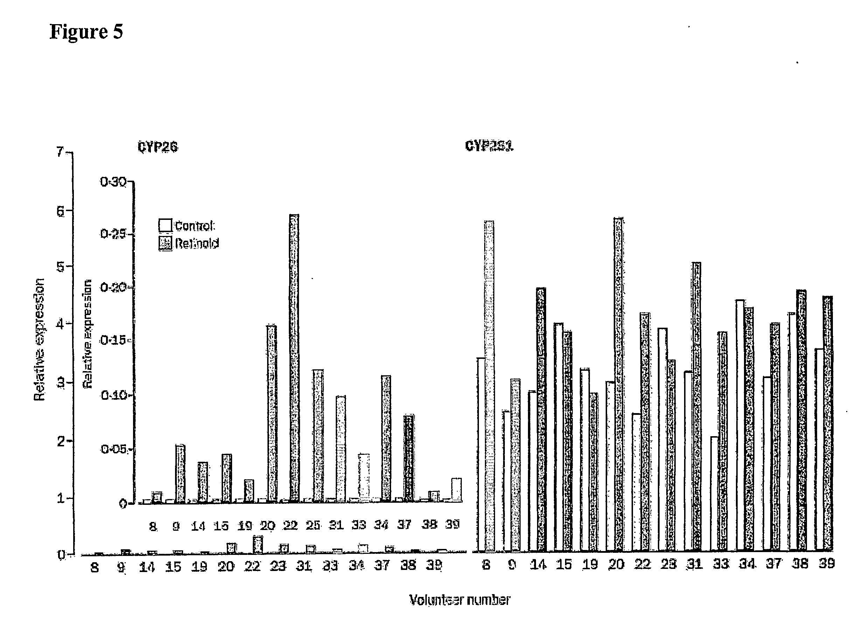

Figure 5 shows the expression of CYP2S1 relative to CYP26 in human skin Quantitative real-time RT-PCR was used to compare CYP2S1 and CYP26 mRNA expression in human skin before and after treatment with atRA. Target gene expression was normalised to 18S ribosomal RNA to ensure equality of loading. All samples were analysed in triplicate; and

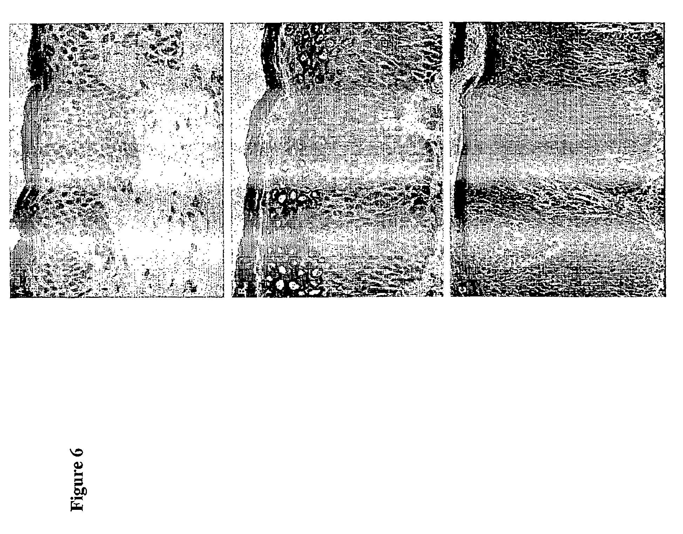

Figure 6 shows CYP2S1 immunohistochemistry
   A: control or untreated skin. B: coaltar treated skin. C: lesional psoriatic skin.
Figure 7 shows 10kb of nucleotide sequence upstream from the ATG stent site for CYP2S1.
   XRE-like sequences are underlined.
   AP-1 like sequences are **in bold**
   RARE-like sequences are *italicised.*

### Methods

### Study participants

Patients with chronic plaque psoriasis, attending the Photobiology Unit, Ninewells Hospital, Dundee, UK, in order to commence UVB phototherapy (n=26) or oral 8-methoxy psoralen photo(chemo)therapy (PUVA) (n=3), were invited to participate in the study. Healthy volunteers (n=27), recruited by local advertising, were invited to participate in the topical chemicals study. All study participants were Caucasian and resident in Tayside at the time of recruitment. Informed consent was obtained from all study participants and the study was approved by the Tayside Committee on Medical Research Ethics.

Irradiation was performed on photoprotected buttock sites of psoriasis patients using either a solar simulator UVR source (150 W xenon arc lamp, 290 - 400 nm, 770 mW/cm², 3.9-48 J/cm²,) (n=26), or a UVA dose series (Waldmann F15W/T8, 320-400 nm, 5-7 mW/cm², 0-5 - 7-9 J/cm²) 2 h after ingestion of 8-methoxypsoralen (0.6 mg/kg, Meladinine^{®}, Galderma, UK) (n=3). At 24 h after solar simulator irradiation or 72 h after PUVA exposure, the minimal dose of radiation required to induce a just perceptible erythema (the minimal erythema dose (MED) or minimal phototoxic dose for PUVA (MPD)) was determined and biopsies performed. Full thickness (4mm diameter) punch biopsies were taken from (a) an irradiated site (1-4 x MED or 3-4 x MPD), (b) adjacent untreated psoriatic plaque and (c) adjacent control site.

To study the effects of topical chemicals, crude coal tar (12% (w/w) in petrolatum) or *all-trans* retinoic acid (atRA) (0·025% (w/w) Retin A^{®} gel) and vehicle control were occluded on aluminium Finn chambers on photoprotected buttock sites of healthy volunteers for 96 hours. After removal of the chambers, full thickness punch biopsies (4 mm diameter) were taken from chemically treated sites and adjacent control skin. All biopsy samples were immediately snap frozen in liquid nitrogen and stored at -70°C prior to analysis.

### RNA preparation and cDNA synthesis

Total RNA was extracted from 4mm diameter skin punch biopsies using Qiagen RNeasy spin columns, according to the manufacturer's instructions, with the addition of Proteinase K and DNase digestion steps. Total RNA from other tissues was prepared using the Promega SV Total RNA isolation system, according to the manufacturer's instructions. RNA concentration and purity was determined spectrophotometrically by measuring fluorescence at 260nM and 280nM. Total RNA (200ng) was reverse transcribed into cDNA in a total volume of 50µl using PE Applied Biosystems Taqman Reverse transcription reagents according to the manufacturer's instructions. 1µl, corresponding to approximately 4 ng of input RNA was used in subsequent Taqman analysis.

### Taqman quantitative real-time PCR analysis

Sequence-specific primers and probes for Taqman quantitative PCR analysis of CYP2S1 mRNA expression were designed using PE Applied Biosystems Primer Express software, according to the manufacturer's protocol. PCR (1 x (50°C, 2 minutes, 95°C, 10 minutes), 40 x (92°C, 15 seconds, 60°C, 1 minute)) was performed in the presence of 0.6 x Taqman Universal PCR Master Mix (PE Applied Biosystems), 300nM forward primer (5'-CGA TGC CTT CCT GCT GAA G-3'), 300nM reverse primer (5'-GCA TGT TCT TGT TGG TGA ATT CTG 3') and 175 nM fluorescent probe (5'-FAM-TGG CAC AGG AGG AAC AAA ACC CAG G-3'). The assay was designed such that the probe spanned an intron/exon boundary to minimise the possibility of co-amplifying genomic DNA. Similarly, assays were designed for CPR (Forward primer 5' CCT GCA GGC CCG CTA CTA 3', Reverse primer 5' TTG GTC TCG TAC TCC ACA ACC A 3', Probe 5'-FAM- TCC TCC AAG GTC CAC CCC AAC TCT GT 3') and CYP26 (Forward primer 5' CCG TAT TTC CTG CGC TTC AT 3', Reverse primer 5' TTC CCC TTC TTT GGG GAA AC 3', Probe 5'-FAM- CAG GAA CTT CCT CCG CTG CAG TAC CAT 3'). Real-time PCR was performed on an ABI Prism 7700 Sequence Detector, where fluorescent output was directly proportional to input cDNA concentration. Input cDNA concentrations were normalised to 18S ribosomal RNA, using PE Applied Biosystems Ribosomal RNA control reagents. Oligonucleotide primers were synthesised by MWG Biotech and fluorescent Taqman probes by PE Applied Biosystems.

### CYP2S1 promoter analysis

To date, the CYP2S 1 cDNA sequence (NCBI Accession number NM_030622) is the only published CYP2S1 sequence information. In order to identify the CYP2S1 promoter, the CYP2S1 cDNA sequence was aligned with a genomic clone (NCBI Accession number NT_011139) containing a region of chromosome 19 encompassing the CYP2 CYP450 cluster¹⁴. A 10kB region immediately upstream of the CYP2S1 transcription start site representing the CYP2S1 gene promoter was analysed for the presence of specific regulatory elements using MatInspector v2·2 software¹⁵.

### atRA metabolism by heterologously expressed CYP2S1

The CYP2S1 cDNA, a generous gift from Professor Oliver Hankinson, Department of Pathology and Laboratory Medicine, Jonsson Comprehensive Cancer Centre and Molecular Biology Institute, UCLA, Los Angeles was constructed and co-expressed with CPR in *E. Coli* as previously described¹⁶. CYP2S1 metabolism of atRA was performed using HPLC analysis as previously described¹⁷, using *E. coli* membranes co-expressing CYP2S1 and CPR and an NADPH regenerating system. Control reactions were performed in the absence of CYP2S1 and in the absence of the NADPH regenerating system. CYP2S1 metabolites were identified by comparison with elution times and UV profiles of atRA metabolites generated by human liver microsomes which had previously been identified using authentic standards.

### Immunohistochemical

4µl frozen sections were cut from 4mm full-thickness skin-biopsy samples that had been taken from photoprotected buttock sites of control or untreated skin, coal tar - treated skin and lesional psoriatic skin. The sections were processed in a standard immunohistochemical procedure with microwave antigen retrieval and a horseradish-peroxidase-labelled strepvidin detection system.

The primary CYP2S1 antibody was raised to a CYP2S1-specific peptide (carboxy terminal 15 aminoacids¹³ and was applied at a dilution of 1 in 500. Sections were counterstained with haematoxylin.

### Statistical analysis

Triplicate measurements of skin mRNA expression were made from each sample, and the mean value taken. After logarithmic transformation, mRNA expression values in control and "lesional" (irradiated, psoriatic plaque or chemically-treated) skin followed a Gaussian distribution, and the paired t-test was used for statistical comparisons of values in "lesional" versus control skin. The possibility of an association between UV dose (in MED-multiples) and relative induction was explored graphically and by Spearman rank correlation, with a confidence interval for the correlation coefficient (rₛ) derived by 'bootstrapping" with 2000 replications. Stata (Intercooled Stata for Windows version 7·0, Stata Corp, Tx, 2002) software was used.

### Results

1. Constitutive CYP2S1 expression in human skin
   The inventors investigated whether CYP2S 1 was expressed in human skin and compared the level of cutaneous expression with other extra-hepatic tissues, including small intestine and kidney, which had previously been reported to express CYP2S1¹³ (Figure 1). They found that extra-hepatic CYP2S1 expression was higher than in human liver. In addition to the small intestine and kidney, CYP2S1 was expressed in breast, placenta, large intestine and human skin. In this initial experiment, CYP2S1 mRNA expression appeared to be induced by UVR exposure and to be elevated in lesional psoriatic skin.
2. Induction of CYP2S1 expression by UVR
   To investigate the effects of UVR exposure on cutaneous CYP2S1 expression, the inventors compared CYP2S1 mRNA levels in non-lesional skin of patients with psoriasis, exposed to a solar simulator at 1-4 x MED UVR with non-lesional untreated skin (n=26) (Figure 2A). CYP2S1 was induced by UVR, with the geometric mean expression 1·78 (95% CI 1·39 to 2·27) times higher in irradiated compared with control skin (p=0·0001). Marked inter-individual variation in both constitutive CYP2S1 expression (up to 5-fold variation) and UVR-inducibility (up to 2-fold variation) was seen. Doses of UVR ranged from 1 x MED to 4 x MED UVR (2-3 x MED in 20/26 patients, Figure 2A). There was no detectable correlation between UVR dose and induction of CYP2S1 in irradiated skin (rₛ=0·30 (95% CI 0-09 to 0.60), p=0·13). Interestingly, the inventors also observed marked induction of CYP2S1 mRNA expression in a smaller number of patients (n=3), 72 hours after exposure to 3-4 x MPD psoralen-UVA (PUVA) irradiation (Figure 2A).
3. Expression of CYP2S 1 in lesional psoriatic skin
   CYP2S1 mRNA expression was compared in biopsies taken from untreated lesional psoriatic skin and adjacent non-lesional control skin (n=29) (Figure 2B). CYP2S1 was consistently higher in psoriatic skin, with the geometric mean expression 3-38 (95% CI 2·64 to 4·34) times higher in psoriatic compared with control skin (p<0·0001). Again, there were significant inter-individual differences in CYP2S 1 inducibility between patients (range 0·58 to 15·53). Interestingly, the inventors also saw marked induction of CPR mRNA levels in psoriatic skin (median induction 3·09, 95% CI 2.30 to 5·11, range 1·23-16-60).
4. CYP2S1 promoter analysis
   In order to investigate possible mechanisms of CYP2S1 regulation, the inventors examined sequences in the CYP2S1 gene promoter to identify regulatory elements which may influence CYP2S1 expression and inducibility. No CYP2S1 promoter sequence has been published to date, but the CYP2S1 gene has been mapped to chromosome 19q13·2¹³. The inventors therefore identified the CYP2S1 gene promoter by mapping the CYP2S1 cDNA onto a genomic clone (NCBI Accession number NT_011139) containing the CYP450 cluster on chromosome 19¹⁴. Recent evidence suggests that transcriptional regulation of CYP450 genes can involve distal regulatory sequences, with a potent enhancer sequence as far as 8kb distal to the transcription start¹⁸. In light of these findings, the inventors analysed a 10kb fragment of the CYP2S1 promoter, including the sequence immediately preceding and including the transcription start site.
   A number of putative transcription factor binding sites were identified, including two sites with complete homology to the AP-1 consensus sequence (5'-TGA(G/C)T(C/A)A-3') and multiple "AP-1 like" sites differing from the AP-1 consensus sequence by a single nucleotide. Interestingly, the inventors also identified several transcription factor binding sites which are known to regulate hepatic CYP450 expression and which may therefore influence cutaneous response to drug and xenobiotic challenge. These include the xenobiotic response element (XRE) which mediates CYP450 induction in response to polycyclic aromatic hydrocarbons¹⁹, and the retinoic acid receptor response element (RARE)²⁰. The CYP2S1 promoter contains 2 XRE sequences (5'-GCGTGCAC-3') identical to the XRE sequences in the major PAH-inducible CYP450s CYP1A1, CYP1A2 and CYP1B1. Retinoic acid receptors (RARs) activate gene transcription in the presence of *all-trans* retinoic acid (atRA) by binding to retinoic acid promoter response elements (RAREs) as heterodimers with retinoid X receptors (RXRs)²¹. RAREs consist of two half-sites with the consensus sequence (5'-AGGTCA-3'), occupied by RAR and RXR respectively, arranged as direct repeats spaced by a single (DR1) or five (DR5) nucleotides²². Multiple RAR consensus half-site sequences are present in the CYP2S1 promoter.
5. Induction of CYP2S1 expression by topical chemicals
   In light of the above analysis, the investigated whether CYP2S1 expression was regulated following the topical application of drugs used routinely in the treatment of psoriasis and other skin diseases. Specifically, they investigated the effects of topically applied crude coal tar (an abundant source of polycyclic aromatic hydrocarbons) and *all-trans* retinoic acid. CYP2S1 mRNA levels were determined following topical drug application and compared with a vehicle control site. The inventors had previously confirmed that, as expected from previous literature²³, coal tar treatment consistently induced, and atRA suppressed CYP1A1 mRNA expression in our volunteer panel, suggesting that both chemicals had been sufficiently absorbed and had been applied at doses which modulated gene transcription (data not shown). Crude coal tar (Figure 3A) and atRA (Figure 3B) were found to induce CYP2S1 mRNA expression, but only in a subset of individuals, with 6/13 coal tar-treated individuals and 6/14 atRA -treated individuals showing marked CYP2S1 induction.
   The inventors confirmed that observed increases in CYP2S1 mRNA expression were reflected an increased protein expression by use of a CYP2S1-specific antibody in immunohistochemical analysis of CYP2S1 expression in control skin, after topical drug treatment, and in lesional psoriatic skin (Figure 6).
6. CYP2S1 makes a significant contribution to cutaneous retinoic acid metabolism CYP2S1 appears to be regulated by atRA in human skin. The observation that CYP450 inducers are also substrates for these enzymes lead the inventors to investigate whether atRA was a CYP2S1 substrate and whether CYP2S1 made a significant contribution to the regulation of cutaneous atRA levels. CYP2S1 was expressed in *E. Coli* together with CPR, using an approach described previously for other CYP450 enzymes¹⁶ and the atRA metabolites produced by heterologously expressed CYP2S1 characterised by HPLC analysis¹⁷. CYP2S1 produced two distinct atRA metabolites which, by comparison with the metabolite profile produced by human liver microsomes, were identified as 4-OH-RA and 5,6-epoxy RA (Figure 4A). In contrast, CYP2S1 did not appear to produce the 4-oxo RA metabolite, which was produced by human liver microsomes (Figure 4B). The identity of this additional metabolite was confirmed using an authentic standard for 4-oxo RA (data not shown).

To determine whether CYP2S1 made a significant contribution to atRA metabolism in human skin, cutaneous CYP2S1 expression was compared to CYP26, a CYP450 which was recently identified as a substrate-inducible and highly specific cutaneous retinoic acid metabolising enzyme²⁴ (Figure 5). Cutaneous CYP26 expression was extremely low compared to CYP2S1, even following induction with atRA.

### Discussion

The difficulty in predicting clinical response or risk of toxicity from topical drug treatment or photo(chemo)therapy is a major clinical problem in the management of common skin diseases, such as psoriasis. The metabolic role of skin is increasingly recognised and inter-individual differences in the cutaneous expression and regulation of drug metabolising enzymes and cytoprotective genes may explain variation in clinical response. Cytochrome CYP450s play a central role in hepatic drug and xenobiotic metabolism, show marked inter-individual differences in catalytic activity and are therefore attractive candidates which may contribute to individuality in cutaneous therapeutic responses.

A number of CYP450 isozymes make a significant contribution to drug metabolism in extra-hepatic tissues¹⁰ and there is mounting evidence that skin, the largest organ of the body, is a metabolically active organ with a diverse and functional CYP450 monoxygenase system^{1-4,13}. The inventors have demonstrated for the first time that a newly identified CYP450, CYP2S1, is expressed in human skin and is inducible by UVR (290-400nm) and PUVA (Figure 2A). Interestingly, analysis of the CYP2S1 promoter revealed the presence of multiple copies of the AP-1 transcription factor binding site, which is known to mediate induction of cutaneous gene transcription in response to a variety of extracellular stimuli including UVR²*⁵.* AP1 binds c-fos/c-jun heterodimers, both of which have been shown to be expressed in the skin²⁶ and, through increased transcription of c-fos and c-jun, is known to mediate UVR induction of the cutaneous expression of genes including the matrix metalloproteinases interstitial collagenase (MMP-1) and stromelysin (MMP-3)²⁷.

Interestingly, the expression of CYP2S1 was significantly elevated in psoriatic plaque. Psoriasis is a chronic hyperproliferative and inflammatory disorder which results in the production of reactive oxygen species (ROS) and proinflammatory cytokines such as interleukin 1 (IL-1) and tumour necrosis factor α (TNFα)²⁸. The marked induction of CYP450 expression in psoriatic skin is of particular interest as the majority of previous literature suggests that CYP450 expression is suppressed in response to inflammatory mediators²⁹. A number of transcription factors including AP-1 and NF-κB have been shown to mediate the effects of inflammatory mediators and ROS on gene transcription^{25,30}. In addition to the AP-1 consensus binding sites described previously, the CYP2S1 gene promoter contains several NF-κB consensus binding sites. The inventors found a highly significant correlation between induction of CYP2S1 mRNA expression by UVR and in psoriatic plaque in individual patients (p=0·003), suggesting that a common mechanism may mediate the effects of ROS and inflammatory mediators on CYP2S1 gene transcription.

CPR was also significantly induced in psoriatic plaque which, in conjunction with the marked increase in CYP2S1 expression, implies a global up-regulation of CYP2S1-mediated metabolism and clearance in lesional psoriatic skin compared with surrounding non-lesional skin. Consistent with this model, it is a common observation in clinical practice that peri-lesional or non-lesional skin of psoriasis patients is more susceptible than lesional psoriatic skin to irritancy following treatment with coal tar or dithranol and is more likely to develop phototherapy-induced erythema.

Significant induction of CYP450 expression in lesional psoriatic skin has implications for the efficacy and tolerability of topical drug therapy, used routinely in the treatment of psoriasis. Based on the relatively high expression of CYP2S1 in skin and its chromosomal localisation in the centre of a cluster of xenobiotic metabolising CYP450s, we would predict that it may play an important role in cutaneous drug metabolism. Topical retinoids are used routinely in the treatment of psoriasis and their metabolism is complex, with the CYP450-catalysed metabolism of at-RA resulting in the formation of many metabolites including 4-OH-RA, 4-oxo-RA, 18-OH-RA and 2,6-epoxy-RA¹⁶. The inventors have shown that cutaneous CYP2S1 expression is significantly higher than CYP26, previously identified as a highly specific and substrate-inducible retinoic acid metabolising enzyme²⁴. The role of CYP26 in retinoic acid metabolism is also described in WO02095034. The inventors have additionally investigated the cutaneous expression and regulation by ATRA of additional CYP450s, including the CYP3A family genes and CYP2C8, which have been shown to contribute to hepatic ATRA metabolism¹⁶. Of these genes, only CYP3A5 was constitutively expressed in human skin and was not inducible by ATRA. In light of the fundamental role played by retinoids in regulating gene expression, in development, and in the maintenance of epithelial tissues, it is proposed that CYP2S1 is an important determinant of cutaneous retinoid homeostasis.

The inventors have also shown that topical coal tar induces cutaneous CYP2S1 expression and identified multiple XRE consensus elements in the CYP2S1 promoter, leading them to predict that PAHs and other CYP1 family substrates would also be metabolised by CYP2S1. It has also been identified that CYP2S1 is a novel dioxin-inducible CYP450³¹.

Interestingly, The inventors saw significant inter-individual differences in CYP2S1 inducibility in response to atRA and coal tar treatment, with a significant proportion of treated individuals showing no induction of CYP2S1 mRNA expression. This result is intriguing as the inventors have previously shown consistent induction of other PAH-inducible CYP450s including CYP1A1 and CYP1B1 by coal tar in the same volunteer panel, suggesting that all the components of the Ah receptor/XRE transcriptional complex are present and functional in all subjects. It appears that inter-individual differences in CYP2S1 inducibility could be explained by genetic polymorphism. The expression of many CYP450s, including CYP1A1, CYP2C19 and CYP2D6, is subject to genetic polymorphism, resulting in a diverse population distribution of alleles, often with significantly different catalytic activities³². CYP2S1 is an obvious target for polymorphism screening and the extent to which genetically determined variation in CYP2S1 expression or inducibility may rationalise inter-individual differences in response to topical drug treatment.

CYP2S1 regulation by UVR, PUVA and by topical drugs used to treat psoriasis raises the possibility that the combination of photo(chemo)therapy and topical drug treatment may have additive or synergistic effects on CYP2S1 expression and inducibility. In support of this hypothesis, there is evidence that the combination of crude coal tar and UVA causes a greater suppression of epidermal DNA synthesis than either single treatment³³ and that enhanced clearance of psoriasis can be achieved by combination therapy with topical coal tar and UVR³⁴.

The inventors have shown that CYP2S1 is expressed in human skin and is induced by UVR, PUVA, selected topical therapeutic chemicals and is elevated in lesional psoriatic skin. The inventors have also shown marked inter-individual differences in response to chemical and UVR exposure. These findings suggest that CYP2S1 may be an important determinant of individuality in cutaneous response to topical drug treatment and photo(chemo)therapy and increase our understanding of the interaction between the skin as a metabolically active organ and the environment.

### REFERENCES

1. Baron JM, Holler D, Schiffer R, Frankenberg S, Neis S, Merk HF, Jugert FK. Expression of multiple cytochrome CYP450 enzymes and multidrug resistance-associated transport proteins in human skin keratinocytes. J Invest Dermatol, 2001; 116: 541-548.
2. Janmohamed A, Dolphin CT, Phillips IR, Shephard EA. Quantification and cellular localization of expression in human skin of genes encoding flavin-containing monooxygenases and cytochromes CYP450. Biochem Pharmacol, 2001; 62: 777-786.
3. Katiyar SK, Matsui MS, Mukhtar H. Ultraviolet-B exposure of human skin induces cytochromes CYP450 1A1 and 1B1. J Invest Dermatol, 2000; 114: 328-333.
4. Gonzalez MC, Marteau C, Franchi J, Migliore-Samour D. Cytochrome P450 4A11 expression in human keratinocytes: effects of ultraviolet irradiation. Br J Dermatol, 2001; 145: 749-757.
5. Wolf CR, Smith G. Pharmacogenetics. Br. Med Bull, 1999; 55,366-386.
6. Savin J, quoting digested statistics from McCormick A, Fleming D, Charlton J. Morbidity statistics from General Practice: 4th National Study 1991-1992. London: HMSO 1995; p54-56
7. Nebert DW. Drug metabolising enzymes, polymorphisms and interindividual response to environmental toxicants. Clin Chem Lab Med, 2000; 38: 857-861.
8. Gibson GG, Skett P. Introduction to drug metabolism, 2nd edition, 1994. Blackie Academic & Professional, Glasgow.
9. Gonzalez FJ, Liu SY, Yano M. Regulation of cytochrome P450 genes: molecular mechanisms. Pharmacogenetics, 1993; 3: 51-57.
10. Tanaka E. Clinically important pharmacokinetic drug-drug interactions: role of cytochrome P450 enzymes. J Clin Pharmacol Ther, 1998; 23: 403-416.
11. Bickers DR, Kappas A. Human skin aryl hydrocarbon hydroxylase - induction by coal tar. J Clin Invest, 1978; 62: 1061.
12. Lawrence CM, Finnen MJ, Shuster S. Effect of coal tar on cutaneous aryl hydrocarbon hydroxylase induction and anthralin irritancy. Br J Dermatol, 1984; 110: 671-675.
13. Rylander T, Neve EPA, Ingelman-Sundberg M, Oscarson M. Identification and tissue distribution of the novel cytochrome P450 CYP2S1 (CYP2S1). Biochem Biophys Res Commun, 2001; 281: 529-535.
14. Hoffman SM, Nelson DR, Keeney DS. Organisation, structure and evolution of the CYP2 gene cluster on chromosome 19. Pharmacogenetics, 2001; 11: 687-698.
15. Wingender E, Chen X, Hehl R, Karas H, Liebich I, Matys V, Meinhardt T, PrüB M, Reuter I, Schacherer F. TRANSFAC: an integrated system for gene expression regulation Nucleic Acids Res. 2000; 28: 316-319.
16. Pritchard MP, Glancey MG, Blake JA, Gilham DE, Burchell B, Wolf CR, Friedberg T. Functional co-expression of CYP2D6 and human NADPH-cytochrome P450 reductase in Escherichia coli. Pharmacogenetics, 1998; 8: 33-42.
17. Marill J, Cresteil T, Lanotte M, Chabot. Identification of human cytochrome P450s involved in the formation of all-trans-retinoic acid principal metabolites. Mol Pharmacol, 2000; 58: 1341-1348.
18. Goodwin B, Hodgson E, Liddle C. The orphan human pregnane X receptor mediates the transcriptional activation of CYP3A4 by rifampicin through a distal enhancer module. Mol Pharmacol, 1999; 56: 1329-1338.
19. Whitlock JP. Induction of cytochrome P4501A1. Ann Rev Pharmacol Toxicol, 1999; 39: 103-125.
20. Glass CK. Some new twists in the regulation of gene expression by thyroid hormone and retinoic acid receptors. J Endocrinol, 1996; 150: 349-357.
21. Chambon P. A decade of molecular biology of retinoic acid receptors. FASEB J, 1996; 10: 195-203.
22. Umesono K, Murakami KK, Thompson CC, Evans RM. Direct repeats as selective response elements for the thyroid hormone, retinoic acid and vitamin D3 receptors. Cell, 1991; 65: 1255-1266.
23. Li XY, Astrom A, Duell EA, Qin L, Griffiths CE, Voorhees JJ. Retinoic acid antagonises basal as well as coal tar and glucocorticoid-induced cytochrome P4501A1 expression in human skin. Carcinogenesis, 1995; 16: 519-524.
24. White JA, Beckett-Jones B, Guo Y-D, Dilworth FJ, Bonasoro J, Jones G, Pelkovich M. cDNA cloning of human retinoic acid-metabolizing enzyme (hP450RAI) identifies a novel family of cytochromes P450 (CYP26). J Biol Chem, 1997; 272: 18538-18541.
25. Karin M, Liu Z, Zandi E. AP-1 function and regulation. Curr Opin Cell Biol, 1997; 9: 240-246.
26. Welter JF, Eckert RL. Differential expression of the fos and jun family members c-fos, fosB, Fra-1, Fra-2, c-jun, junB and junD during human epidermal keratinocyte differentiation. Oncogene, 1995; 11: 2681-2687.
27. Fisher GJ, Wang ZQ, Datta SC, Varani J, Kang S, Voorhees JJ. Pathophysiology of premature skin aging induced by ultraviolet light. N Engl J Med, 1997; 337: 1419-1428.
28. Allen RG, Tresini, M. Oxidative stress and gene regulation. Free Rad Biol Med, 2000; 28: 463-499.
29. Morgan ET. Regulation of cytochrome P450 by inflammatory mediators: why and how? Drug Metab Dispos, 2001; 29: 207-212.
30. Bäuerle PA, Henkel T. Function and activation of NFκB in the immune system. Ann Rev Immunol, 1994; 12: 141-179.
31. Rivera SP, Saarikoski ST, Hankinson O. Identification of a novel dioxin-inducible cytochrome P450. Mol Pharmacol, 2002; 61: 255-259.
32. Wolf CR, Smith G, Smith RL. Science, medicine and the future: Pharmacogenetics. BMJ, 2000; 320: 987-990.
33. Stoughton RB, Dequoy P, Walter JF. Crude coal tar plus near ultraviolet light suppresses DNA synthesis in epidermis. Arch Dermatol, 1978; 114: 43-45.
34. Fischer T. Comparative treatment of psoriasis with UV-light, trioxsalen plus UV-light and coal tar plus UV-light. Acta Derm Venereol, 1977; 57: 345-350.

## Claims

1. A method for identifying an agent capable of modulating expression of CYP2S1 by a skin cell, comprising the steps of:
a) providing a skin cell or cells capable of expressing CYP2S1;
b) contacting a test agent with said cell(s);
c) incubating said ccll(s) under conditions which are conducive to enable expression of CYP2S1 when in the absence of the test agent; and
d) detecting whether or not the test agent modulates expression of CYP2S1.

2. A method for identifying an agent capable of modulating expression of CYP2S 1 by a skin cell, comprising the steps of:
a) providing a skin cell comprising the sequence, as shown in Figure 7 or a fragment thereof capable of controlling transcription and/or translation of a reporter nucleic acid located downstream thereof;
b) contacting a test agent with said cell(s);
c) incubating said cell(s) under conditions which are conducive to enable transcription and/or translation of said reporter nucleic acid located downstream; and
d) detecting whether or not the test agent modulates transcription and/or translation of said reporter nucleic acid.

3. The method according to claim 2 wherein the reporter nucleic acid is capable of encoding glutathione S-transferase, an antibiotic, a chromogenic substrate, such as β-galactocidase, luciferase, a fluorescent protein, such as green fluorescent protein, or chloramphenicol acetyl transferase.

4. The method according to any preceding claim wherein said skin cell(s) is/are a keratinocyte and/or an epidermal cell(s).

5. The method according to any preceding claim wherein the test agent is an antisense oligonucleotide, and RNAi molecule, a chemical drug candidate, a peptide or protein, radiation, such as UV radiation or radiation in combination with a chemical which is capable of being activated by said radiation.

6. The method according to any preceding claim, wherein the test agent is capable of increasing expression and/or activity of CYP2S1.

7. The method according to any one of claims 1 to 5 wherein the test agent is capable of decreasing expression and/or activity of CYP2S1.

8. The method according to any preceding claim for identifying an agent capable of modulating CYP2S1 expression and/or activity for use in treating a skin disorder.

9. The method according to claim 8 wherein the skin disorder is psoriasis.

10. The method according to claim 1 wherein detection of any modulation in the expression of CYP2S1 is carried out using an antibody specifically reactive to CYP2S1.

11. The method according to claim 1 wherein detection of any modulation in the expression of CYP2S1 mRNA is carried out using quantitative real time PCR analysis.

12. A skin cell comprising a recombinant vector comprising a nucleic acid capable of encoding CYP2S1 or a receptor protein under transcriptional and/or translational control of an isolated nucleic acid molecule comprising the sequence of Figure 7 or fragment thereof.

13. The skin cell according to claim 12 wherein the reporter protein is glutathione S-transferase, an antibiotic, a chromogenic substrate, such as β-galactocidase, luciferase, a fluorescent protein, such as green fluorescent protein, chloramphenicol acetyl transferase.

14. Use of the host cell of claims 12 or 13 in a method according to any one of claims 1 to 11.

15. Use of CYP2S 1 in the manufacture of a medicament for treating a skin disorder associated with a decrease in CYP2S1 expression in skin, or for administering to a subject displaying reduced CYP2S1 expression.

16. A method useful in diagnosing a skin condition associated with increased or decreased expression of CYP2S1, or a predisposition to such a condition comprising detecting a level of CYPZS1 in a test skin sample and comparing this against a normal control such that an increase or decrease in CYP2S1 expression in the test sample as compared to the normal control is indicative of a skin condition or predisposition to a skin condition.

17. A method useful in diagnosing a skin condition associated with increased or decreased expression of CYP2S 1 or a predisposition to such a condition comprising detecting a polymorphism in a CYP2S1 gene or upstream sequence thereof, which effects expression of CYP2S 1, wherein detection of a polymorphism is indicative of a skin disorder associated with increased or decreased CYP2S1 expression, or predisposition thereto.

18. An *in vitro* method of detecting effectiveness of a skin treatment to be administered to a patient suffering from a skin condition, comprising the steps of:
a) providing a sample of diseased skin and detecting a level of CYP2S1 expression in the sample of diseased skin prior to administration of the skin treatment; and
b) providing a further sample of diseased skin after administration of the skin treatment and detecting whether or not there has been an increase or decrease in CYP2S1 expression.

19. An *in vitro* method of detecting whether or not a subject is likely to respond to a skin treatment comprising the step of:
detecting a level of CYP2S1 expression in samples of diseased and non-diseased skin from a subject wherein an increase in expression of CYP2S1 in a diseased skin sample, relative to a non-diseased skin sample, is indicative of a subject who may respond favourably to a chemical which is metabolisable by CYP2S1.

## Patentansprüche

1. Verfahren zur Ermittlung eines Wirkstoffs, der fähig ist, die Expression von CYP2S1 durch eine Hautzelle zu modulieren, wobei das Verfahren die folgenden Schritte aufweist:
a) Bereitstellen einer oder mehrerer Hautzellen mit der Fähigkeit, CYP2S 1 zu exprimieren;
b) Inkontaktbringen eines Testwirkstoffs mit der (den) Zelle(n);
c) Inkubieren der Zelle(n) unter Bedingungen, die dazu beitragen, in Abwesenheit des Testwirkstoffs die Expression von CYP2S1 zu ermöglichen; und
d) Feststellen, ob der Testwirkstoff die Expression von CYP2S1 moduliert oder nicht.

2. Verfahren zur Ermittlung eines Wirkstoffs, der fähig ist, die Expression von CYP2S1 durch eine Hautzelle zu modulieren, wobei das Verfahren die folgenden Schritte aufweist:
a) Bereitstellen einer Hautzelle, welche die in Fig. 7 dargestellte Sequenz oder ein Fragment davon aufweist, die (das) fähig ist, die Transkription und/oder Translation einer stromabwärts davon gelegenen Reporter-Nucleinsäure zu steuern;
b) Inkontaktbringen eines Testwirkstoffs mit der (den) Zelle(n);
c) Inkubieren der Zelle(n) unter Bedingungen, die dazu beitragen, die Transkription und/oder Translation der stromabwärts gelegenen Reporter-Nucleinsäure zu ermöglichen; und
d) Feststellen, ob der Testwirkstoff die Transkription und/oder Translation der Reporter-Nucleinsäure moduliert oder nicht.

3. Verfahren nach Anspruch 2, wobei die Reporter-Nucleinsäure fähig ist, Glutathion-S-Transferase, ein Antibiotikum, ein chromogenes Substrat, wie z. B. β-Galactosidase, Luciferase, ein fluoreszierendes Protein, wie z. B. grün fluoreszierendes Protein, oder Chloramphenicolacetyltransferase zu codieren.

4. Verfahren nach einem der vorstehenden Ansprüche, wobei die eine oder die mehreren Hautzellen Keratinozyten oder Epidermiszellen sind.

5. Verfahren nach einem der vorstehenden Ansprüche, wobei der Testwirkstoff ein Antisense-Oligonucleotid, ein RNAi-Molekül, ein Kandidat für ein chemisches Medikament, ein Peptid oder Protein, Strahlung, wie z. B. UV-Strahlung, oder Strahlung in Kombination mit einer Chemikalie ist, die durch die Strahlung aktiviert werden kann.

6. Verfahren nach einem der vorstehenden Anspruche, wobei der Testwirkstoff fähig ist, die Expression und/oder Aktivität von CYP2S 1 zu erhöhen.

7. Verfahren nach einem der Anspruche 1 bis 5, wobei der Testwirkstoff fähig ist, die Expression und/oder Aktivität von CYP2S1 zu vermindern.

8. Verfahren nach einem der vorstehenden Ansprüche zur Ermittlung eines Wirkstoffs, der fähig ist, die Expression und/oder Aktivität von CYP2S1 zur Verwendung bei der Behandlung einer Hauterkrankung zu modulieren.

9. Verfahren nach Anspruch 8, wobei die Hauterkrankung Psoriasis ist.

10. Verfahren nach Anspruch 1, wobei der Nachweis irgendeiner Modulation bei der Expression von CYP2S1 mit einem spezifisch CYP2S1-reaktiven Antikörper geführt wird.

11. Verfahren nach Anspruch 1, wobei der Nachweis irgendeiner Modulation bei der Expression von CYP2S1-mRNA unter Anwendung der quantitativen Echtzeit-PCR-Analyse geführt wird.

12. Hautzelle, die einen rekombinanten Vektor mit einer Nucleinsäure aufweist, die fähig ist, CYP2S1 oder ein Reporterprotein unter Transkriptions- und/oder Translationssteuerung eines isolierten Nucleinsäuremoleküls zu codieren, das eine Sequenz gemäß Fig. 7 oder ein Fragment davon aufweist,

13. Hautzelle nach Anspruch 12, wobei das Reporterprotein Glutathion-S-Transferase, ein Antibiotikum, ein chromogenes Substrat, wie z. B. β-Calactosidase, Luciferase, ein fluoreszierendes Protein, wie z. B. grün fluoreszierendes Protein, oder Chloramphenicolacetyltransferase ist.

14. Verwendung einer Wirtszelle nach Anspruch 12 oder 13 in einem Verfahren nach einem der Absprüche 1 bis 11.

15. Verwendung von CYP2S1 bei der Herstellung eines Medikaments zur Behandlung einer Hauterkrankung, die mit einer Verminderung der CYP2S1-Expression in der Haut verbunden ist, oder zur Verabreichung an einen Patienten, der verminderte CYP2S1-Expression aufweist.

16. Verfahren, das bei der Diagnose eines Hautzustands, der mit erhöhter oder verminderter CYP2S1-Expression verbunden ist, oder einer Prädisposition zu einem solchen Zustand anwendbar ist, wobei das Verfahren die Bestimmung eines CYP2S1-Spiegels in einer Hauttestprobe und den Vergleich dieses Spiegels mit einer normalen Kontrolle aufweist, so daß eine Erhöhung oder Verminderung der CYP2S1-Expression in der Testprobe im Vergleich zu der normalen Kontrolle auf einen Hautzustand oder eine Prädisposition zu einem Hautzustand schließen läßt.

17. Verfahren, das bei der Diagnose eines Hautzustands, der mit erhöhter oder verminderter CYP2S1-Expression verbunden ist, oder einer Prädisposition zu einem solchen Zustand anwendbar ist, wobei das Verfahren den Nachweis eines Polymorphismus in einem CYP2S1-Gen oder einer stromaufwärts gelegenen Sequenz davon aufweist, der eine Expression von CYP2S1 bewirkt, wobei der Nachweis eine Polymorphismus auf eine Hauterkrankung, die mit erhöhter oder verminderter CYP2S1-Expression verbunden ist, oder auf eine Prädisposition dazu schließen läßt.

18. In-vitro-Verfahren zum Nachweis der Wirksamkeit einer Hautbehandlung, die bei einem Patienten durchgeführt werden soll, der an einer Hauterkrankung leidet, mit den folgenden Schritten:
a) Bereitstellen einer Probe der erkrankten Haut und Bestimmen einer CYP2S1-Expressionstärke in der Probe der erkrankten Haut vor Durchführung der Hautbehandlung; und
b) Bereitstellen einer weiteren Probe der erkrankten Haut nach Durchführung der Hautbehandlung und Feststellen, ob eine Erhöhung oder Verminderung der CYP2S1-Expression stattgefunden hat oder nicht.

19. In-vitro-Verfahren, um festzustellen, ob ein Patient wahrscheinlich auf eine Hautbehandlung ansprechen wird oder nicht, wobei das Verfahren den folgenden Schritt aufweise
Bestimmen eines CYP2S1-Expressionsspiegels in Proben von erkrankter und nicht erkrankter Haut von einem Patienten, wobei eine Erhöhung der CYP2S1-Expression in einer erkrankten Hautprobe gegenüber einer nicht erkrankten Hautprobe auf einen Patienten schließen läßt, der unter Umständen günstig auf eine Chemikalie anspricht, die durch CYP2S 1 metabolisierbar ist.

## Revendications

1. Procédé pour l'identification d'un agent capable de moduler l'expression de CYP2S1 par une cellule de peau, comprenant les étapes:
a) de fourniture d'une cellule ou de cellules de peau capables d'exprimer CYP2S1;
b) de mise en contact d'un agent d'essai avec ladite ou lesdites cellules;
c) d'incubation de ladite ou desdites cellules sous des conditions qui contribuent à permettre l'expression de CYP2S1 en l'absence de l'agent d'essai; et
d) de détection si oui ou non l'agent d'essai module l'expression de CYP2S1.

2. Procédé pour l'identification d'un agent capable de moduler l'expression de CYP2S1 par une cellule de peau, comprenant les étapes:
a) de fourniture d'une cellule de peau comprenant la séquence telle que montrée dans la Figure 7 ou un fragment de celle-ci capable de contrôler la transcription et/ou la traduction d'un acide nucléique rapporteur localisé en aval de celle-ci;
b) de mise en contact d'un agent d'essai avec ladite ou lesdites cellules;
c) d'incubation de ladite ou desdites cellules sous des conditions qui contribuent à permettre la transcription et/ou la traduction dudit acide nucléique rapporteur localisé en aval; et
d) de détection si oui ou non l'agent d'essai module la transcription et/ou la traduction dudit acide nucléique rapporteur.

3. Procédé suivant la revendication 2, dans lequel l'acide nucléique rapporteur est capable de coder une glutathion-S-transférase, un antibiotique, un substrat chromogéne, tel qu'une β-galactosidase, une luciférase, une protéine fluorescente, telle qu'une protéine fluorescente verte, ou une chloramphénicol-acétyl-transférase.

4. Procédé suivant l'une quelconque des revendications précédentes, dans lequel ladite ou lesdites cellules est/sont une ou des cellules de kératinocyte et/ou épidermiques,

5. Procédé suivant l'une quelconque des revendications précédentes, dans lequel l'agent d'essai est un oligonucléotide anti-sens, et une molécule RNAi, un candidat de médicament chimique, un peptide ou une protéine, un rayonnement, tel qu'un rayonnement UV ou un rayonnement en combinaison avec un composé chimique qui est capable d'être activé par ledit rayonnement

6. Procédé suivant l'une quelconque des revendications précédentes, dans lequel l'agent d'essai est capable d'augmenter l'expression et/ou l'activité de CYP2S1.

7. Procédé suivant l'une quelconque des revendications 1 à 5, dans lequel l'agent d'essai est capable de diminuer l'expression et/ou l'activité de CYP2S1.

8. Procédé suivant l'une quelconque des revendications: précédentes pour l'identification d'un agent capable de moduler l'expression et/ou l'activité de CYP2S1 pour une utilisation dans le traitement d'un trouble de la peau.

9. Procédé suivant la revendication 8, dans lequel le trouble de la peau est un psoriasis.

10. Procédé suivant la revendication 1, dans lequel la détection de toute modulation dans l'expression de CYP2S1 est réalisée en utilisant un anticorps spécifiquement réactif à CYP2S1.

11. Procédé suivant la revendication 1, dans lequel la détection de toute modulation dans l'expression d'un ARNm de CYP2S1 est réalisée en employant une analyse PCR quantitative en temps réel.

12. Cellule de peau comprenant un vecteur recombinant comprenant un acide nucléique capable de coder CYP2S1 ou une protéine réceptrice sous un contrôle de transcription et/ou de traduction d'une molécule d'acide nucléique isolée comprenant la séquence de la Figure 7 ou un fragment de celle-ci.

13. Cellule de peau suivant la revendication 12, dans laquelle la protéine rapporteuse est une glutathion-S-transférase, un antibiotique, un substrat chromogène, tel qu'une β-galactosidase, une luciférase, une protéine fluorescente, telle qu'une protéine fluorescente verte, une chloramphénicol-acétyl-transférase.

14. Utilisation de la cellule hôte suivant la revendication 12 ou 13 dans un procédé suivant l'une quelconque des revendications 1 à 11.

15. Utilisation de CYP2S1 dans la fabrication d'un médicament pour le traitement d'un trouble de la peau associé à une diminution dans l'expression de CYP2S1 dans la peau, ou pour une administration à un sujet affichant une expression de CYP2S1 réduite.

16. Procédé utile dans le diagnostic d'un état de la peau associé à une expression augmentée ou diminuée de CYP2S1, ou d'une prédisposition à un tel état comprenant la détection d'un niveau de CYP2S 1 dans un échantillon de peau d'essai et la comparaison de celui-ci vis-à-vis d'un témoin normal de sorte qu'une augmentation ou une diminution de l'expression de CYP2S1 dans l'échantillon d'essai lorsqu'il est comparé au témoin normal est une indication d'un état de la peau ou d'une prédisposition à un état de la peau.

17. Procédé utile dans le diagnostic d'un état de la peau associé à une expression augmentée ou diminuée de CYP2S1 ou d'une prédisposition à un tel état comprenant la détection d'un polymorphisme dans un gène de CYP2S1 ou une séquence en amont de celui-ci, qui l'expression de CYP2S1, dans lequel la détection d'un polymorphisme est une indication d'un trouble de la peau associé à une expression de CYP2S1 augmentée ou diminuée ou d'une prédisposition à celui-ci.

18. Procédé in vitro pour la détection de l'efficacité d'un traitement de la peau à administrer à un patient souffrant d'un état de la peau, comprenant les étapes:
a) de fourniture d'un échantillon d'une peau malade et de détection d'un niveau d'expression de CYP2S1 dans l'échantillon de peau malade avant l'administration du traitement de la peau; et
b) de fourniture d'un échantillon supplémentaire de peau malade après l'administration du traitement de la peau et de détection si oui ou non il y a eu une augmentation ou une diminution dans l'expression de CYP2S 1.

19. Procédé in vitro pour la détection si oui ou non un sujet est susceptible de répondre à un traitement de la peau comprenant l'étape:
de détection d'un niveau d'expression de CYP2S1 dans des échantillons de peau malade et non malade provenant d'un sujet, dans lequel une augmentation dans l'expression de CYP2S1 dans un échantillon de peau malade, relativement à un échantillon de peau non malade, est une indication d'un sujet qui peut répondre favorablement à un composé chimique qui est métabolisable par GYP2S1.
